# EUROPÄISCHE PATENTSCHRIFT

(11) **EP 3 644 946 B1**
(45) Veröffentlichungstag und Bekanntmachung des Hinweises auf die Patenterteilung: **19.03.2025**
(21) Anmeldenummer: 18727262.0
(22) Anmeldetag: 24.05.2018
(51) Int. Cl.: A61K 8/365, A61K 8/362, A61K 8/22, A61Q 5/08

(54) **VERFAHREN ZUM AUFHELLEN VON HAAREN ENTHALTEND SPEZIELLE OXO-CARBONSÄUREN**
PROCESS FOR LIGHTENING HAIR, CONTAINING SPECIAL OXO-CARBOXYLIC ACIDS
PROCÉDÉ POUR ÉCLAIRCIR LES CHEVEUX CONTENANT DES ACIDES OXO-CARBOXYLIQUES SPÉCIAUX

(30) Priorität: 27.06.2017 DE 102017210809
(43) Veröffentlichungstag der Anmeldung: 06.05.2020
(73) Patentinhaber: Henkel AG & Co. KGaA, 40589 Düsseldorf (DE)
(72) Erfinder: GEBERT-SCHWARZWAELDER, Antje, 41469 Neuss (DE); KOENEN, Annika, 41516 Grevenbroich (DE); KROOS, Astrid, 40789 Monheim (DE)
(86) Internationale Anmeldenummer: PCT/EP2018/063617
(87) Internationale Veröffentlichungsnummer: WO 2019/001856

(56) Entgegenhaltungen:
- EP-A1- 3 181 113
- EP-A1- 3 287 120
- DE-U1- 202016 008 131
- JP-A- 2014 080 384
- US-A1- 2012 204 357

## Beschreibung

Die vorliegende Erfindung liegt auf dem Gebiet der Kosmetik. Gegenstand der vorliegenden Erfindung ist ein Verfahren zum Aufhellen von keratinischen Fasern, insbesondere menschlichen Haaren, bei dem eine Mehrkomponenten-Verpackungseinheit, welche getrennt konfektioniert mindestens ein erstes Mittel mit einer Peroxid-Verbindung und mindestens ein zweites Mittel mit einer speziellen Oxo-Carbonsäure umfasst, zum Einsatz kommt.

Beim oxidativen Färben oder Blondieren von Haaren tritt das Problem auf, dass es aufgrund der aggressiven Agentien zu Schäden an der keratinischen Faser kommen kann. Insbesondere die natürliche Hydrophobizität der keratinischen Faser wird reduziert, da die Färbe- bzw. Aufhellmittel das Haar zunächst penetrationsfähig machen müssen, um ihre Wirkung zu entfalten. Die wasserabweisende Wirkung ist aber einerseits ein natürlicher Schutz des Haares, andererseits sind vom Verbraucher erwünschte Parameter wie Glanz, Geschmeidigkeit, Haarfestigkeit, Griff und "Fallen" der Haare eng mit ihr verknüpft.

Um die genannten Nachteile zu überwinden, ist aus dem Stand der Technik der Einsatz von sogenannten Keratinvernetzern bekannt. Bei den Keratinvernetzern handelt es sich um ungesättigte, monomere Verbindungen mit einer Molmasse von weniger als 500 g/mol. Werden die Keratin-vernetzer nun auf Haaren angewendet, so können sie aufgrund ihrer kleinen Molekülmasse gut in die Haarfaser hinein diffundieren. Im Inneren der Haarfaser bilden die Keratinvernetzer dann - entweder direkt mit der Haarfaser oder mit anderen Vernetzer-Molekülen - Addukte aus, wobei diese Addukt-Bildung an der Doppelbindung eines jeden Vernetzer-Moleküls erfolgt.

Entsprechende Keratinvernetzer werden beispielsweise in EP 2478892 A1 beschrieben. Chemisch gesehen handelt es sich bei dieser Vernetzung um die Addition an eine Doppelbindung. Um die Doppelbindung des Keratinvernetzers zu aktivieren, befindet sich diese oft in direkter Nachbarschaft zu einer stark elektronenziehenden Gruppe (wie beispielweise einer Carboxylgruppe). Zusätzlich kann die Additionsreaktion auch noch durch den Einsatz von Initiatoren erleichtert werden. In EP 2478892 A1 können als Initiatoren beispielsweise auch Persulfate, Persäuren oder AzoVerbindungen eingesetzt werden. Als optimaler pH-Wert für diese Reaktion wird in diesem Zusammenhang ein saurer Bereich von 4,0 bis 6,9 beschrieben.

In den Arbeiten, die dieser Anmeldung zugrunde lagen, wurde nun jedoch herausgefunden, dass die in EP 2478892 A1 beschriebenen Keratin-Vernetzer auf die Blondierung bzw. Aufhellung von Keratinfasern nicht optimal zugeschnitten sind.

Die Einstellung eines alkalischen pH-Wertes ist zur Erzielung einer ausreichend hohen Blondierwirkung unumgänglich. Wird der in EP 2478892 A1 vorgeschlagene saure pH-Wert eingestellt, so ist die Blondierleistung viel zu schwach.

JP 2014080384 A betrifft eine auf zwei Mitteln basierende Haarbehandlung, wobei das erste Mittel Cystin (A) und Alkalisierungsmittel (B), und das zweite Mittel eine organische Säure (C) beinhaltet. Der pH-Wert des ersten Mittels liegt bei 8,5 - 11, und der pH-Wert des zweiten Mittels liegt im Bereich von 3 - 5.

EP 3181113 A1 beschreibt eine Haarfärbezusammensetzung, enthaltend mindestens ein Acrylsäure-Derivat einer Formel (I) und mindestens ein Oxidationsfarbstoffvorprodukt. Der pH-Wert der Haarfärbe-zusammensetzung liegt bei 8 - 12.

EP 3287120 A1 betrifft ein Haarfärbeverfahren, wobei eine erste Zusammensetzung und eine zweite Zusammensetzung auf die Haare appliziert werden. Die zweite Zusammensetzung enthält mindestens eine Säure einer Formel (II) und mindestens eine weitere Säure einer Formel (III).

In DE 20 2016 008131 U1 wird ein Kit zur Haarbehandlung beschrieben, der zwei getrennte Formulierungen umfasst, wobei die erste Formulierung eine bifunktionelle Brönsted-Base enthält und einen pH-Wert von 7 -12 besitzt, und die zweite Formulierung eine bifunktionelle Brönsted-Säure enthält und einen pH-Wert von 1,7 bis 7 aufweist.

US 2012/204357 A1 beschreibt ein Färbemittel, enthaltend einen hydrophoben Direktfarbstoff mit einem log P-Wert von mehr als 2, eine organische Carbonylverbindung und eine weitere organische Verbindung mit einem bestimmten Hansen-Löslichkeitsparameter.

Der vorliegenden Erfindung lag deshalb die Aufgabe zugrunde, ein Mittel zur oxidativen Aufhellung bzw. Blondierung von Haaren bereitzustellen, das die Haare nicht bzw. möglichst wenig schädigt, aber dennoch eine sehr starke Aufhell-Leistung besitzt.

Dabei sollten insbesondere Aufhellmittel bereitgestellt werden, durch welche das Haar stark blondiert werden kann, die Haare aber dennoch nicht brüchig, glanzlos oder auf eine andere Weise geschädigt werden. Trotz starker Aufhellung sollte die gesamte Haarfaser stabilisiert werden. Weiterhin sollte der erzielte Haarschutz möglichst wenig zeitaufwändig sein und möglichst zusammen mit dem Färbe- bzw. Aufhellschritt selbst erfolgen.

Es wurde nun gefunden, dass die vorgenannte Aufgabe in vollem Umfang gelöst werden kann, wenn für die Aufhellung bzw. Blondierung eine Mehrkomponenten-Verpackungseinheit eingesetzt wird, welche getrennt konfektioniert in zwei Containern mindestens zwei Mittel (A) und (B) umfasst.

Das Mittel (A), welches sich im ersten Container befindet, stellt eine Oxidationsmittel-Zubereitung dar und enthält mindestens eine Peroxid-Verbindung, bevorzugt Wasserstoffperoxid. Das zweite Mittel (B) beinhaltet mindestens eine Oxo-Carbonsäure einer speziellen Formel (I).

Ein erster Gegenstand der vorliegenden Erfindung ist ein Verfahren zum Aufhellen von menschlichen Haaren, umfassend die folgenden Schritte in der angegebenen Reihenfolge
(1) Vermischen eines Mittels (A) mit einem Mittel (C) und/oder einem Mittel (D), wobei
   - das Mittel (A)
      (a) Wasserstoffperoxid, enthält, und
         - das Mittel (C) mindestens ein Persulfat aus der Gruppe aus Ammoniumpersulfat, Kaliumpersulfat und/oder Natriumpersulfat enthält, und
         - das Mittel (D) mindestens ein Alkalisierungsmittel aus der Gruppe aus Ammoniak, 2-Aminoethan-1-ol, 2-Amino-2-methylpropan-1-ol, 2-Amino-2-methyl-propan-1,3-diol, L-Arginin, D-Arginin, D/L-Arginin, L-Lysin, D-Lysin und D/L-Lysin enthält,
(2) Auftragen der in Schritt (1) hergestellten Mischung auf die Haare,
(3) Einwirkenlassen der in Schritt (2) applizierten Mischung auf den Haaren,
(4) gegebenenfalls Ausspülen der Haare,
(5) Applizieren eines Mittels (B) auf die Haare, wobei
   - das Mittel (B)
      (b) mindestens eine Säure der Formel (I) enthält, 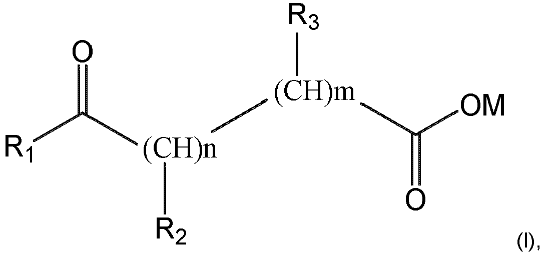 wobei
      - R₁: für eine C₁-C₆-Alkylgruppesteht,
      - R₂, R₃: unabhängig voneinander für ein Wasserstoffatom, eine C₁-C₆-Alkylgruppe odereine C₂-C₆-Alkenylgruppe, stehen,
      - M: für ein Wasserstoffatom oder ein Äquivalent eines ein- oder mehrwertigen Kations steht,
      - n: für die Zahl 1 oder 2 steht und
      - m: für die Zahl 0 oder 1 steht,
      und wobei das Mittel (B) Wasser enthält und einen pH-Wert von 1,8 bis 3,0 besitzt.

Es konnte festgestellt werden, dass durch Anwendung der Mehrkomponenten-Verpackungseinheit im erfindungsgemäßen Verfahren beim anschließenden Kämmen weniger Haarbruch auftrat und die Stabilität der Faser erhöht war. Die Erhöhung der Faserstabilität ließ sich beispielweise mittels DSC (Difference Scanning Calorimetrie) Messungen feststellen. Weiterhin büßte das Haar weniger an Elastizität ein als nach der Applikation nicht erfindungsgemäßer Blondiermittel. Die Elastizität eines Haares konnte beispielsweise durch Zug-Dehnungs-Messungen gemessen werden.

Unter dem erfindungsgemäß verwendeten Begriff "Aufhellen von keratinischen Fasern" wird insbesondere eine Blondierung oder Bleiche der Fasern verstanden. Nach der Anwendung des Aufhellmittels besitzt die behandelte Keratinfaser einen helleren Farbton als vor Anwendung des Aufhell- oder Blondiermittels. Die Stärke der Aufhellung kann beispielsweise visuell beurteilt oder auch mittels farbmetrischer Vermessung der Haarsträhne quantifiziert werden (Messung der Lab-Werte). Bei der farbmetrischen Vermessung gibt der L-Wert die Helligkeit einer Keratinfaser bzw. Haarsträhne an (bei L=100 ist die Haarsträhne diffus weiß, bei L=0 ist die Haarsträhne schwarz). Nach der Anwendung des erfindungsgemäßen Aufhellmittels hat die Strähne demnach einen höheren L-Wert.

Unter dem erfindungsgemäß verwendeten Begriff "Aufhellen von keratinischen Fasern" wird erfindungsgemäß auch eine aufhellende Färbung (oder färbende Blondierung) verstanden. In diesem Fall kann das Mittel zusätzlich zur Peroxid-Verbindung auch Farbstoffe enthalten, wobei das Mittel diese Farbstoffe jedoch nur in geringen Mengen zur Nuancierung des Aufhellergebnisses enthält. Nach Anwendung eines farbstoffhaltigen Aufhellmittels besitzt die behandelte Keratinfaser deshalb ebenfalls einen helleren Farbton als vor der Anwendung des Mittels.

Die im erfindungsgemäßen Verfahren eingesetzten Mittel (A) und (B) enthalten die erfindungswesentlichen Bestandteile (a) und (b) jeweils in einem kosmetischen Träger. Zum Zwecke der Aufhellung (bzw. aufhellenden Färbung) sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind. Besonders bevorzugt sind die kosmetischen Träger der Mittel (A) und/oder (B) wässrig oder wässrig-alkoholisch.

Unter wässrig-alkoholischen Lösungen sind im Sinne der vorliegenden Erfindung insbesondere wässrige Lösungen enthaltend 0,1 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

### Mittel (A)

Als erfindungswesentlichen Bestandteil (a) enthält das Mittel (A) mindestens eine Peroxid-Verbindung. Bei der Peroxid-Verbindung (a) handelt es sich um das Oxidationsmittel, welche die Aufhellung, Blondierung bzw. Bleiche der Keratinfasern bewirkt. Bei der Peroxid-Verbindung handelt es sich um Wasserstoffperoxid.

Wasserstoffperoxid wird entweder in Form seiner wässrigen Lösung und/oder in Form eines seiner festen Anlagerungsprodukte an organische oder anorganische Verbindungen, wie Harnstoff, Melamin sowie Natriumborat eingesetzt. Ganz besonders bevorzugt wird Wasserstoffperoxid in Form seiner wässrigen Lösung eingesetzt.

Bevorzugt beträgt die Menge an Wasserstoffperoxid im Mittel (A) 0,1 bis 12,5 Gew.-%, bevorzugt 1,5 bis 12,5 Gew.-%, weiter bevorzugt 4,5 bis 12,5 Gew.-% und ganz besonders bevorzugt 6,5 bis 12,5 Gew.-% Wasserstoffperoxid (berechnet als 100 %-iges H₂O₂), jeweils bezogen auf das Gesamtgewicht des Mittels (A).

In einer besonders bevorzugten Ausführungsform ist einerfindungsgemäßes Verfahren dadurch gekennzeichnet, dass das Mittel (A) - bezogen auf das Gesamtgewicht des Mittels (A) - 0,1 bis 12,5 Gew.-%, bevorzugt 1,5 bis 12,5 Gew.-%, weiter bevorzugt 4,5 bis 12,5 Gew.-% und ganz besonders bevorzugt 6,5 bis 12,5 Gew.-% Wasserstoffperoxid (a) enthält.

### Mittel (B)

Als erfindungswesentlichen Bestandteil (b) enthält das Mittel (B) mindestens eine Säure der Formel (I), 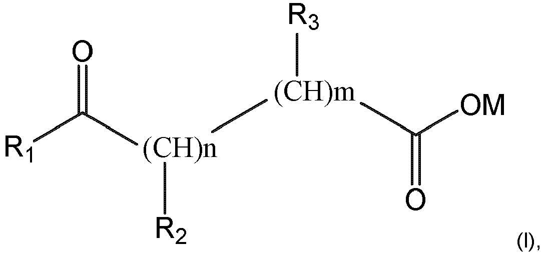 wobei
- R₁: für eine C₁-C₆-Alkylgruppe, steht,
- R₂, R₃: unabhängig voneinander für ein Wasserstoffatom, eine C₁-C₆-Alkylgruppe oder eine C₂-C₆-Alkenylgruppe stehen,
- M: für ein Wasserstoffatom oder ein Äquivalent eines ein- oder mehrwertigen Kations steht,
- n: für die Zahl von 1 oder 2 steht und
- m: für die Zahl von 0 oder 1 steht.

Es hat sich herausgestellt, dass die erfindungsgemäßen Säuren der Formel (I) die Schädigung der Keratinfasern effektiv vermindern, wenn sie während oder nach einer Blondierung auf die Keratinfasern appliziert werden.

Messungen, in denen die Wirkungen der erfindungsgemäßen Säuren der Formel (I) mit der Wirkung anderer, nicht erfindungsgemäße Säuren verglichen wurden, zeigten, dass die Säuren der Formel (I) bei gleichem pH-Wert eine stark verbesserte Strukturierung und einen erhöhten Schutz der Keratinfaser bewirkten und die durch die Blondierung hervorgerufene Schädigung der Keratinfaser wirkungsvoll minimierten.

Im Folgenden werden Beispiele für die in Formel (I) genannten Substituenten R₁, R₂ und R₃ exemplarisch genannt: Beispiele für C₁-C₆-Alkylgruppen sind -CH₃, -CH₂CH₃, -CH₂CH₂CH₃, - CH(CH₃)₂, -CH₂CH₂CH₂CH₃, -CH₂CH(CH₃)₂, -CH(CH₃)CH₂CH₃, -C(CH₃)₃, -(CH₂)₄CH₃, -(CH₂)₅CH₃. Besonders bevorzugte Alkylreste sind Methyl und Ethyl. Beispiele für C₂-C₆-Alkenylgruppen sind Vinyl, Prop-2-enyl (Allyl), 2-Methyl-prop-2-enyl, But-3-enyl, But-2-enyl, Pent-4-enyl oder Pent-3-enyl.

Bei den Carbonsäuren der Formel (I) steht der Rest R₁ für eine C₁-C₆-Alkylgruppe.

In diesem Zusammenhang wurde herausgefunden, dass mit den Säuren der Formel (I) die beste Schutzwirkung erzielt werden konnte, bei denen R₁ für eine C₁-C₆-Alkylgruppe, bevorzugt für eine Methylgruppe, eine Ethylgruppe oder eine Propylgruppe, und ganz besonders bevorzugt für eine Methylgruppe steht.

In einer besonders bevorzugten Ausführungsform ist ein erfindungsgemäßes Verfahren dadurch gekennzeichnet, dass das Mittel (B) mindestens eine Säure der Formel (I) enthält, wobei
- R₁: für eine Methylgruppe, eine Ethylgruppe oder eine Propylgruppe und besonders bevorzugt für eine Methylgruppe, steht.

Bei den Carbonsäuren der Formel (I) können die Reste R₂ und R₃ unabhängig voneinander gewählt werden. Die Reste R₂, R₃ stehen unabhängig voneinander für ein Wasserstoffatom, eine C₁-C₆-Alkylgruppe oder eine C₂-C₆-Alkenylgruppe

Es hat sich weiterhin herausgestellt, dass die Carbonsäuren der Formel (I) zur Lösung der erfindungsgemäßen Aufgabenstellung ganz besonders gute Eignung zeigen, wenn die Reste R₂ und R₃ unabhängig voneinander für ein Wasserstoffatom, eine C₁-C₆-Alkylgruppe oder eine C₂-C₆-Alkenylgruppe stehen.

In einer weiteren besonders bevorzugten Ausführungsform ist eine erfindungsgemäßes Verfahren dadurch gekennzeichnet, dass das Mittel (B) mindestens eine Säure der Formel (I) enthält, wobei
- R₂, R₃: beide für ein Wasserstoffatom stehen.

In den Verbindungen der Formel (I) gibt n die Anzahl der Wiederholungeinheiten für die Gruppierung (CHR₂) an. Analog gibt m die Anzahl der Wiederholungeinheiten für die Gruppierung (CHR₃) an.

Steht n beispielsweise für die Zahl 1, und m für die Zahl 1 so handelt es sich bei der Säure der Formel (I) um die Verbindung R₁C(O)-CHR₂-CHR₃-COOM.

Steht n beispielsweise für die Zahl 2, und m für die Zahl 1 so handelt es sich bei der Säure der Formel (I) um die Verbindung R₁C(O)-CHR₂-CHR₂-CHR₃-COOM.

Das erfindungsgemäße Verfahren ist dadurch gekennzeichnet, dass das Mittel (B) eine Säure der Formel (I) enthält, wobei
- n: für die Zahl 1 oder 2 steht und
- m: für die Zahl 0 oder 1 steht.

In den Verbindungen der Formel (I) steht M für ein Wasserstoffatom oder ein Äquivalent eines ein-oder mehrwertigen Kations steht.

Wenn M für ein Wasserstoffatom steht, so liegt die Verbindung der Formel (I) in Form ihrer Säure vor. Das Wasserstoffatom der Säurefunktion ist acide, und in wässrigem Medium wird die Säure deprotoniert. Aus diesem Grund liegt die Säure (I) im kosmetischen Mittel (B) - insbesondere wenn dieses einen wasserhaltigen kosmetischen Träger umfasst - im Gleichgewicht mit ihrem Anion vor.

Die Säure kann auch direkt in Form ihres Salzes im kosmetischen Mittel (B) eingesetzt werden. In diesem Fall wird das Carboxylat-Anion durch M neutralisiert, wobei M ein Äquivalent eines ein- oder mehrwertigen Kations steht. Als geeignete anorganische Kationen sind insbesondere Na⁺, K⁺, (NH₄)⁺, ½ Mg²⁺, ½ Ca²⁺ zu nennen. Auch organische Kationen wie Tetra-C₁-C₂₂-Alkylammonium sind geeignet.

Das Mittel (B) enthält Wasser und wird auf einen sauren pH-Wert eingestellt. Die Einstellung des sauren pH-Wertes kann beispielsweise dadurch erfolgen, dass die Säure(n) der Formel (I) in Form ihrer freien Säure eingesetzt werden. Demzufolge hat es sich als ganz besonders bevorzugt herausgestellt, wenn M für ein Wasserstoffatom steht.

In einer besonders bevorzugten Ausführungsform ist ein erfindungsgemäßes Verfahren dadurch gekennzeichnet, dass das Mittel (B) mindestens eine Säure der Formel (I) (bzw. (la)) enthält, wobei
- M: für ein Wasserstoffatom steht.

Erfindungsgemäß bevorzugte Mittel (B) zum Aufhellen von keratinischen Fasern sind dadurch gekennzeichnet, dass sie mindestens eine Carbonsäure Formel (I) enthalten, die ausgewählt ist aus
- 4-Oxopentansäure (Lävulinsäure) 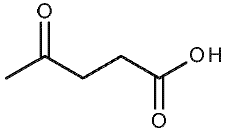
- 4-Oxohexansäure 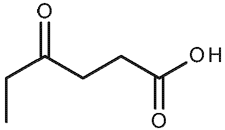
- 2-Methyl-4-oxo-pentansäure 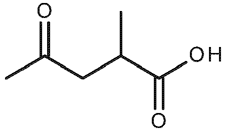
- 3-Methyl-4-oxo-pentansäure

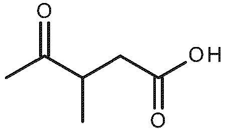

Innerhalb der vorgenannten Gruppe der bevorzugten Carbonsäure Formel (I) nochmals explizit ganz besonders bevorzugt ist die Verbindung
- 4-Oxopentansäure (Lävulinsäure).
4-Oxopentansäure (Lävulinsäure) ist kommerziell erwerblich.

Zur Erzielung eines optimalen Faserschutzes enthalten die erfindungsgemäßen Mittel (B) den oder die Säuren der Formel (I) bevorzugt in bestimmten Mengenbereichen. Ganz besonders bevorzugt ist es, wenn das erfindungsgemäße Mittel (B) - bezogen auf sein Gesamtgewicht - (b) ein oder mehrere Säuren der Formel (I) in einer Gesamtmenge von 0,1 bis 10,0 Gew.-%, bevorzugt von 0,3 bis 8,5 Gew.-%, weiter bevorzugt von 0,5 bis 7,5 Gew.-% und ganz besonders bevorzugt von 0,7 bis 6,5 Gew.-% enthält.

In einer besonders bevorzugten Ausführungsform ist ein erfindungsgemäßes Verfahren dadurch gekennzeichnet, dass das Mittel (B)- bezogen auf das Gesamtgewicht des Mittels (B) - ein oder mehrere Säuren der Formel (I) in einer Gesamtmenge von 0,1 bis 10,0 Gew.-%, bevorzugt von 0,3 bis 8,5 Gew.-%, weiter bevorzugt von 0,5 bis 7,5 Gew.-% und ganz besonders bevorzugt von 0,7 bis 6,5 Gew.-% enthält.

### pH-Wert des Mittels (B)

Wie bereits zuvor beschrieben, werden ganz besonders bevorzugt die Säuren der Formel (I) im erfindungsgemäßen Mittel (B) eingesetzt, bei denen M für ein Wasserstoffatom steht, d.h. als besonders vorteilhaft hat sich der Einsatz der Säuren in ihrer freien Form erwiesen. Die Acidität der Säuregruppe trägt dazu bei, dass der pH-Wert des Mittels (B) im sauren Bereich liegt. Besonders bevorzugt enthält das Mittel (B) Wasser.

Das Mittel (B) wird auf einen pH-Wert im Bereich von 1,8 bis 3,0 eingestellt.

Die Messung des pH-Wertes kann beispielsweise mit einer Glaselektrode erfolgen, die üblicherweise in Form einer Einstabmesskette kommerziell erwerblich ist. Vor der Messung des pH-Wertes werden die Glaselektroden üblicherweise mit Kalibrierlösungen bekannten pH-Wertes kalibriert. Unter den pH-Werten im Sinne der vorliegenden Erfindung werden pH-Werte verstanden, die bei einer Temperatur von 22 °C gemessen wurden.

Zur Einstellung der bevorzugten pH-Werte können insbesondere die Säuren der Formel (I) eingesetzt werden. Zusätzlich können auch weitere Mengen auch Acidifizierungsmittel im Mittel (B) enthalten sein. Erfindungsgemäß geeignete weitere Acidifizierungsmittel sind beispielsweise Zitronensäure, Milchsäure, Essigsäure, Weinsäure, Äpfelsäure, Etidronsäure, Pyridin-2,6-dicarbonsäure oder auch verdünnte Mineralsäuren (wie beispielsweise Salzsäure, Schwefelsäure, Phoshporsäure).

Zur Feinjustierung des pH-Wertes können darüberhinaus auch verschiedene Alkalisierungsmittel im Mittel (B) verwendet werden. Erfindungsgemäß geeignete Alkalisierungsmittel können ausgewählt werden aus der Gruppe, die gebildet wird aus Ammoniak, Alkanolaminen, Alkalimetallhydroxiden, Alkalimetallmetasilikaten, Alkalimetallphosphaten und Alkalimetallhydrogenphosphaten. Bevorzugte anorganische Alkalisierungsmittel sind Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Natriumsilicat und Natriummetasilicat sind ganz besonders bevorzugt.

### Mittel (C)

Die Mittel (B), welche die erfindungsgemäßen Säuren der Formel (I) enthalten, eignen sich hervorragend zur Reduzierung der Schädigungen, die durch die Bleiche und Blondierung von Haaren hervorgerufen werden. Die Blondierung von Haaren wird hierbei durch die Mittel (A), welche mindestens ein Peroxid, bevorzugt Wasserstoffperoxid enthalten, bewirkt.

Wünscht der Anwender eine besonders starke Aufhellleistung, so reicht Wasserstoffperoxid als alleiniges Oxidationsmittel in der Regel nicht aus. In diesem Fall werden üblicherweise Blondiermittel eingesetzt, welche die Kombination der Oxidationsmittel Wasserstoffperoxid und Persulfaten enthalten.

Im Zuge der zu dieser Erfindung führenden Arbeiten hat sich herausgestellt, dass die Mittel (B) mit den Säuren der Formel (I) auch bei bzw. insbesondere nach Einsatz der Kombination Wassrstoffperoxid/Persulfat eine signifikante Reduzierung der Schädigung bewirken.

Aus diesem Grund ist es besonders bevorzugt, das Mittel (B) mit den Säuren der Formel (I) nach einer Blondierung anzuwenden, bei der die Haare mittels einer Kombination von Wasserstoffperoxid und Persulfaten blondiert wurden. Zu diesem Zweck wird im erfindungsgemäßen Verfahren ganz besonders bevorzugt ein drittes Mittel (C) angewendet. Hierbei enthält dieses dritte Mittel (C) Ammoniumpersulfat, Kaliumpersulfat und/oder Natriumpersulfat.

Persulfate werden alternativ auch als Peroxodisulfate bezeichnet. Geeignete Persulfate sind Ammoniumpersulfat (Ammoniumperoxodisulfat), Kaliumpersulfat (Kaliumperoxodisulfat) und Natriumpersulfat (Natriumperoxodisulfat).

Kaliumperoxodisulfat wird alternativ auch als Kaliumpersulfat bezeichnet und besitzt die Summenformel K₂S₂O_{8.}

Ammoniumperoxodisulfat wird alternativ auch als Ammoniumpersulfat bezeichnet und besitzt die Summenformel (NH₄)₂S₂O₈.

Natriumperoxodisulfat wird alternativ auch als Natriumpersulfat bezeichnet und besitzt die Summenformel Na₂S₂O₈.

Das oder die Persulfate werden bevorzugt in einer Gesamtmenge von 1,0 bis 40,0 Gew.-%, bevorzugt von 5,0 bis 30,0 Gew.-% weiter bevorzugt von 10,0 bis 25 Gew.-% und ganz besonders 15,0 bis 20,0 Gew.-% im Mittel (C) eingesetzt, wobei die vorgenannten Mengenangaben auf die Gesamtmenge der im Mittel (C) eingesetzten Persulfate bezogen sind, die zum Gesamtgewicht des Mittels (C) in Relation gesetzt wird.

### pH-Wert des Blondiermittels

Die Bleiche oder Blondierung von Haaren findet üblicherweise unter alkalischen Bedigungungen statt. Das Alkalisierungsmittel sorgt für eine ausrechende Quellung der Haare, auch die Blondierleistung von Wasserstoffperoxid bzw. Wasserstoffperoxid/Persulfat steigt im alkalischen Milieu. Aus diesem Grunde besitzt das Anwendungsbereite Blondiermittel üblicherweise einen pH-Wert von 7,5 bis 12,5, bevorzugt von 8,5 bis 10.5.

Aus Stabilitätsgründen wird das Mittel (A), welches Wasserstoffperoxid enthält, sauer eingestellt. Aus diesem Grund wird ein Alkalisierungsmittel daher üblicherweise nicht zusammen mit dem Wasserstoffperoxid konfektioniert.

Zur Herstellung eines alkalischen Blonidermittels kann das Alkalisierungsmittel daher beispielsweise in das Mittel (C) eingearbeitet werden. Das Mittel (C) enthält Persulfate und wird bevorzugt in Form eines Pulvers oder einer Paste zur Verfügung gestellt.

Der Einsatz von Alkalisierungsmitteln im Mittel (C) ist deshalb insbesondere für die Alkalisierungsmittel gut geeignet, die ebenfalls in fester bzw. pulverförmiger Form vorliegen. Geeignete feste Alkalisierungsmittel sind beispielsweise Alkalimetallhydroxide, Alkalimetallmetasilikate, Alkalimetallphosphate und Alkalimetallhydrogenphosphate. Bevorzugte anorganische Alkalisierungsmittel sind Natriumhydroxid, Kaliumhydroxid, Natriumsilicat und Natriummetasilicat. Natriumsilicat und Natriummetasilicat sind ganz besonders bevorzugt.

Unter Natriumsilicaten werden die Natriumsalze verschiedener Kieselsäuren verstanden. Unterschieden werden können Natriumsilicate, bei denen das Verhältnis von Siliciumdioxid zu Natriummonoxid größergleich 2 oder kleinergleich 1 ist. Zur letzteren Gruppe gehört das Natriummetasilikat, d.h. unter Natriummetasilicat werden polymere Silicate der Formel [Na₂SiO₃]ₓ verstanden. Natriummetasilikat kann wasserfrei oder auch in Form seiner Hydrate eingesetzt werden.

Weiterhin kann das Natriumsilicat auch in Form von Natriumwasserglas eingesetzt werden. Bei Natriumwasserglas handelt es sich um das aus einer Schmelze erstarrte, amorphe Natriumsilikat.

Bevorzugte anorganische Alkalisierungsmittel sind Natriumhydroxid, Kaliumhydroxid, Natriumsilicat und Natriummetasilicat.

Andererseits kann das Blondiermittel auch durch Einsatz von flüssigen oder fließbaren Alkalisierungsmittel auf den optimalen pH-Wert eingestellt werden. Als geeignete flüssige oder fließbare Alkalisierungsmittel können Ammoniak, 2-Aminoethan-1-ol, 2-Amino-2-methylpropan-1-ol, 2-Amino-2-methyl-propan-1,3-diol genannt werden.

Auch Amionosäuren wie L-Arginin, D-Arginin, D/L-Arginin, L-Lysin, D-Lysin und D/L-Lysin sind als Alkalisierungsmittel geeignet.

Die vorgenannten Alkalisierungsmittel können in einem weiteren Container bereitgestellt werden, der ein weiteres Mittel (D) enthält. Im Rahmen dieser Ausführungsform enthält das Mittel (D) mindestens ein Alkalisierungsmittel aus der Gruppe aus Ammoniak, 2-Aminoethan-1-ol, 2-Amino-2-methyl-propan-1-ol, 2-Amino-2-methyl-propan-1,3-diol , L-Arginin, D-Arginin, D/L-Arginin, L-Lysin, D-Lysin und D/L-Lysin.

### Weitere Inhaltsstoffe

Die Mittel (A) und (B) (sowie gegebenenfalls die Mittel (C) und/oder (D)) können zusätzlich weitere Wirk-, Hilfs- und Zusatzstoffe enthalten, wie beispielsweise kationische Tenside, nichtionische Tenside, amphotere bzw. zwitterionische Tenside, anionische Tenside, anionische, nichtionische und/oder kationische Polymere, Strukturanten wie Glucose, Parfümöle, faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Farbverändernde Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Vitamine, Provitamine und Vitaminvorstufen; Pflanzenextrakte; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft.

Die zusätzlichen Wirk- und Hilfsstoffe werden in den erfindungsgemäßen Mitteln bevorzugt in Mengen von jeweils 0,0001 bis 10 Gew.-%, insbesondere von 0,0005 bis 5 Gew.-%, bezogen auf das Gesamtgewicht des jeweiligen Mittels eingesetzt.

### Verfahren zur Blondierung von Haaren

Das Verfahren zum Aufhellen von Haaren, umfasst die folgenden Schritte in der angegebenen Reihenfolge
(1) Vermischen eines Mittels (A) mit einem Mittel (C) und/oder einem Mittel (D), wobei
   - es sich bei dem Mittel (A) um ein Mittel handelt, wie es bereits im Detail offenbart wurde und
   - es sich bei dem Mittel (C) um ein Mittel handelt, wie es bereits im Detail b offenbart wurde, und
   - es sich bei dem Mittel (D) handelt, wie es bereits im Detail offenbart wurde,
(2) Auftragen der in Schritt (1) hergestellten Mischung auf die Haare,
(3) Einwirkenlassen der in Schritt (2) applizierten Mischung auf den Haaren,
(4) gegebenenfalls Ausspülen der Haare,
(5) Applizieren eines Mittels (B) auf die Haare, wobei es sich bei dem Mittel (B) um ein Mittel der handelt, wie es bereits im Detail offenbart wurde.

Das Mittel (A) enthält Wasserstoffperoxid und stellt die Oxidationsmittelkomponente dar. Zur Herstellung des anwendungsbereiten Blondiermittels wird das Mittel (A) - abhängig vom gewünschten Aufhellgrad - entweder mit dem Mittel (C) und/oder mit dem Mittel (D) vermischt, wobei das Mittel (C) ein oder mehrere Persulfate und das Mittel (D) ein oder mehrere Alkalisierungsmittel enthält (Schritt (1)).

Die Mischung der Mittel (A) und (C) stellt ein anwendungsbereites Blondiermittel dar.

Die Mischung der Mittel (A) und (D) stellt ebenfalls ein anwendungsbereites Blondiermittel dar. Auch die Mischung der Mittel (A), (C) und (D) stellt ein anwendungsbereites Blondiermittel dar.

So können beispielsweise
- 100 g des Mittels (A) mit 100 g des Mittels (C) vermischt werden oder auch
- 100 g des Mittels (A) mit 100 g des Mittels (D) vermischt werden oder auch
- 100 g des Mittels (A) mit 100 g des Mittels (C) und mit 100 g des Mittels (D) vermischt werden oder auch
- 100 g des Mittels (A) mit 50 g des Mittels (C) und mit 50 g des Mittels (D) vermischt werden

Auch andere Mischungsverhältnisse sind erfindungsgemäß und möglich. So können die Mittel (A) und (C) im Mengenverhältnis 1:5 bis 5:1, bevorzugt 1:3 bis 1:3 miteinander vermischt werden.

Die Mittel (A) und (D) können beispielsweise im Mengenverhältnis 1:5 bis 5:1, bevorzugt 1:3 bis 1:3 miteinander vermischt werden.

Diese in Schritt (1) hergestellte Mischung, bei der es sich um das anwendungsbereite Blondiermittel handelt, wird nun auf die Haare aufgetragen (Schritt 2). Die Auftragung kann hierbei mit der behandschuhten Hand oder aber mit Hilfe eines Applikators (Kamm, Bürste oder Applicette) erfolgen. Nach dem Auftragen wird das anwendungsbereite Blondiermittel auf die Haare einwirken gelassen, beispielsweise für einen Zeitraum von 5 bis 60 Minuten, bevorzugt von 15 bis 45 Minuten (Schritt 3).

Nun kann das Blondiermittel von den Haaren ausgespült werden (Schritt 4).

Als besonders bevorzugt hat es sich herausgestellt, die Haare für einen kurzen Zeitraum von 5 bis 60 Sekunden, bevorzugt von 5 bis 45 Sekunden auszuspülen.

Besonders bevorzugt ist ein Verfahren zum Aufhellen von Haaren, umfassend die folgenden Schritte in der angegebenen Reihenfolge
(1) Vermischen eines Mittels (A) mit einem Mittel (C) und/oder einem Mittel (D), wobei
   - es sich bei dem Mittel (A) um ein Mittel handelt, wie es bereits im Detail offenbart wurde und
   - es sich bei dem Mittel (C) um ein Mittel handelt, wie es bereits im Detail offenbart wurde, und
   - es sich bei dem Mittel (D) handelt, wie es bereits im Detail offenbart wurde,
(2) Auftragen der in Schritt (1) hergestellten Mischung auf die Haare,
(3) Einwirkenlassen der in Schritt (2) applizierten Mischung auf den Haaren,
(4) Ausspülen der Haare für einen Zeitraum von 5 bis 60, bevorzugt von 5 bis 45 Sekunden,
(5) Applizieren eines Mittels (B) auf die Haare, wobei es sich bei dem Mittel (B) um ein Mittel der handelt, wie es bereits im Detail offenbart wurde.

Im Rahmen einer weiteren Ausführungsform kann das Mittel (B) aber auch ohne Ausspülen auf die noch mit Blondiermittel beaufschlagten Haare appliziert werden.

Geeignet ist auch ein Verfahren zum Aufhellen von Haaren, umfassend die folgenden Schritte in der angegebenen Reihenfolge
(1) Vermischen eines Mittels (A) mit einem Mittel (C) und/oder einem Mittel (D), wobei
   - es sich bei dem Mittel (A) um ein Mittel handelt, wie es bereits im Detail offenbart wurde und
   - es sich bei dem Mittel (C) um ein Mittel handelt, wie es bereits im Detail offenbart wurde, und
   - es sich bei dem Mittel (D) handelt, wie es bereits im Detail offenbart wurde,
(2) Auftragen der in Schritt (1) hergestellten Mischung auf die Haare,
(3) Einwirkenlassen der in Schritt (2) applizierten Mischung auf den Haaren,
(4) Applizieren eines Mittels (B) auf die Haare, wobei es sich bei dem Mittel (B) um ein Mittel der handelt, wie es bereits im Detail offenbart wurde.

Das Mittel (B), das die Säure(n) der Formel (I) enthält, wird im erfindungsgemäßen Verfahren als Nachbehandlungsmittel appliziert, welches nach dem Auftragen des anwendungsbereiten Blondiermittels auf die Haare appliziert wird.

Besonders bevorzugt liegt das Mittel (B) als wasserhaltiges kosmetisches Mittel vor, das einen sauren pH-Wert besitzt und beipsielsweise als Gel, Shampoo, Conditioner oder als Creme konfektioniert werden kann.

### Beispiele:

### 1. Formulierungen

Es wurden die folgenden Formulierungen hergestellt (alle Angaben in Gew.-%)

### Formulierung mit Wasserstoffperoxid (Mittel (A))

| Substanz (INCI) | OX |
|---|---|
| Na-benzoate | 0,04 |
| Dipicolinic acid | 0,10 |
| Disodium pyrophosphate | 0,10 |
| Potassium hydroxide 50% | 0,19 |
| Propanediol-1,2 | 0,50 |
| HEDP 60% | 0,25 |
| Paraffinum Liquidum | 2,00 |
| Cetearyl Alcohol | 3,60 |
| Ceteareth-20 | 1,20 |
| Hydrogen peroxide 50 % | 12,20 |
| Water, demineralized | ad 100 |

### Paste mit Persulfat (Mittel (C))

| Substanz (INCI) | V1 |
|---|---|
| Versagel M1600 | 5,00 |
| Lanette N | 7,00 |
| Eumulgin B 5 | 4,00 |
| Xanthan NaTrue | 1,50 |
| Sodium metasilicate (wasserfrei) | 6,50 |
| Potassium persulfate | 42,00 |
| Parfum | 0,60 |
| Paraffinum Liquidum | ad 100 |

| | |
|---|---|
| Versagel M1600: | Paraffinum Liquidum (Mineral Oil), Ethylene/Propylene/Styrene Copolymer, Butylene/Ethylene/Styrene Copolymer |
| Lanette N: | Cetearyl Alcohol, Sodium Cetearyl Sulfate |
| Eumulgin B 5: | Ceteareth-50 |
| Xanthan NaTrue: | Xanthan Gum |

### Nachbehandlungsmittel (B)

| | (B1) | (B2) | (B3) | (B4) | (B5) | (B6) |
|---|---|---|---|---|---|---|
| | E | V | E | V | E | V |
| Lävulinsäure | 1,0 | --- | 5,0 | --- | 10,0 | --- |
| Salzsäure | --- | ad pH 2,7 | --- | ad pH 2,3 | --- | ad pH 2,2 |
| pH-Wert | 2,7 | 2,7 | 2,3 | 2,3 | 2,2 | 2,2 |
| Wasser | ad 100 | ad 100 | ad 100 | ad 100 | ad 100 | ad 100 |

### Lävulinsäure (Alternativnamen: Levulinsäure, 4-Oxo-pentansäure, CAS-Nr. 123-76-2, Merck)

| | (B7) | (B8) | (B9) | (B10) | (B11) | (B12) |
|---|---|---|---|---|---|---|
| | E | V | V | V | V | V |
| Lävulinsäure | 3,0 | --- | --- | --- | --- | --- |
| Itaconsäure | --- | --- | 0,14 | --- | 0,56 | --- |
| Salzsäure | --- | ad 2,4 | --- | ad pH 2,7 | --- | ad pH 2,3 |
| pH-Wert | 2,4 | 2,4 | 2,7 | 2,7 | 2,3 | 2,3 |
| Wasser | ad 100 | ad 100 | ad 100 | ad 100 | ad 100 | ad 100 |

### Itaconsäure (Alternativnamen: Methylensuccinsäure, 1-Propen-2,3-dicarbonsäure, CAS-Nr. 97-65-4, Merck)

Als Vergleich (V) diente jeweils ein Nachbehandlungsmittel, das keine erfindungsgemäße Säure der Formel (I) enthielt, sondern bei dem so viel Salzsäure zugegeben wurde, bis die Vergleichsformulierung denselben pH-Wert wie das entsprechende erfindungsgemäße Nachbehandlungsmittel (E) hatte.

### 2. Anwendung

Die Wasserstoffperoxid-Formulierung (Mittel A) und die Persulfat-Paste (Mittel C) wurde jeweils im Verhältnis 1:1 vermischt und sofort nach dem Vermischen auf jeweils 12 Haarsträhnen aufgetragen (Einwirkzeit 45 min bei Raumtemperatur). Der pH-Wert der Anwendungsmischung lag jeweils bei 9,5. Die Strähnen wurden nach Beendigung der Einwirkzeit für 10 Sekunden mit Leitungswasser ausgespült.

Die als Referenz (R) dienende Strähne wurde nicht mit einem Mittel (B) nachbehandelt, sondern stattdesssen gründlich mit Leitungswasser ausgespült. Im Anschluss daran wurden die Strähnen für jeweils 10 Minuten in jeweils eines der Mittel (B) getaucht, im Anschluss daran gründlich mit Wasser ausgespült und getrocknet.

### 3. Messung der Faserstabilisierung

Mittele einer DSC-Analyse (Perkin Elmer DSC-7) wurden die folgenden Schmelzpunkte ermittelt. Eine genaue Beschreibung der Methode findet sich beispielsweise in DE 196 173 95 A1.

Je höher der Messwert (Peak Apex Temp, °C) ist, desto stabiler ist die Keratinmatrix des Haares.

### Haarstabilität (12-fach Bestimmung mit Mittelwertbildung)

| | (R) ohne | (B1) | (B2) | (B3) | (B4) | (B5) | (B6) |
|---|---|---|---|---|---|---|---|
| | Nachbehandlung | E | V | E | V | E | V |
| DSC: Peak Apex Temp. (°C) | 143 | 159 | 147 | 163 | 151 | 164 | 157 |

| | (R) ohne | (B7) | (B8) | (B9) | (B10) | (B11) | (B12) |
|---|---|---|---|---|---|---|---|
| | Nachbehandlung | E | V | V | V | V | V |
| DSC: Peak Apex Temp. (°C) | 143 | 161 | 150 | 159 | 147 | 162 | 151 |

## Patentansprüche

1. Verfahren zum Aufhellen von Haaren, umfassend die folgenden Schritte in der angegebenen Reihenfolge
(1) Vermischen eines Mittels (A) mit einem Mittel (C) und/oder einem Mittel (D), wobei
- das Mittel (A) Wasserstoffperoxid enthält, und
- das Mittel (C) mindestens ein Persulfat aus der Gruppe aus Ammoniumpersulfat, Kaliumpersulfat und/oder Natriumpersulfat enthält, und
- das Mittel (D) mindestens ein Alkalisierungsmittel aus der Gruppe aus Ammoniak, 2-Aminoethan-1-ol, 2-Amino-2-methylpropan-1-ol, 2-Amino-2-methyl-propan-1,3-diol , L-Arginin, D-Arginin, D/L-Arginin, L-Lysin, D-Lysin und D/L-Lysin enthält,
(2) Auftragen der in Schritt (1) hergestellten Mischung auf die Haare,
(3) Einwirkenlassen der in Schritt (2) applizierten Mischung auf den Haaren,
(4) gegebenenfalls Ausspülen der Haare,
(5) Applizieren eines Mittels (B) auf die Haare, wobei das Mittel (B) mindestens eine Säure der Formel (I) enthält, 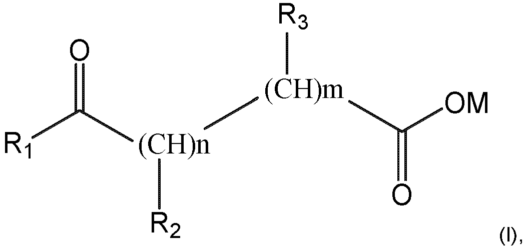 wobei
R₁ für eine C₁-C₆-Alkylgruppe steht,
R₂, R₃ unabhängig voneinander für ein Wasserstoffatom, eine C₁-C₆-Alkylgruppe, eine C₂-C₆-Alkenylgruppe stehen,
M für ein Wasserstoffatom oder ein Äquivalent eines ein- oder mehrwertigen Kations steht,
n für die Zahl 1 oder 2 steht und
m für die Zahl 0 oder 1 steht,
und wobei das Mittel (B) Wasser enthält und einen pH-Wert von 1,8 bis 3,0 besitzt.

2. Verfahren nach Anspruch 1, **dadurch gekennzeichnet, dass** das Mittel (A) - bezogen auf das Gesamtgewicht des Mittels (A) - 0,1 bis 12,5 Gew.-%, bevorzugt 1,5 bis 12,5 Gew.-%, weiter bevorzugt 4,5 bis 12,5 Gew.-% und ganz besonders bevorzugt 6,5 bis 12,5 Gew.-% Wasserstoffperoxid enthält.

3. Verfahren nach einem der Ansprüche 1 bis 2, **dadurch gekennzeichnet, dass** das Mittel (B) mindestens eine Säure der Formel (I) enthält, wobei
R₁ für eine Methylgruppe, eine Ethylgruppe oder eine Propylgruppe und bevorzugt für eine Methylgruppe, steht.

4. Verfahren nach einem der Ansprüche 1 bis 3, **dadurch gekennzeichnet, dass** das Mittel (B) mindestens eine Säure der Formel (I) enthält, wobei
M für ein Wasserstoffatom steht.

5. Verfahren nach einem der Ansprüche 1 bis 4, **dadurch gekennzeichnet, dass** das Mittel (B) mindestens eine Säure der Formel (I) enthält, die ausgewählt ist aus der Gruppe aus 4-Oxopentansäure (Lävulinsäure), 4-Oxohexansäure, 2-Methyl-4-oxo-pentansäure und/oder 3-Methyl-4-oxo-pentansäure.

6. Verfahren nach einem der Ansprüche 1 bis 5, **dadurch gekennzeichnet, dass** das Mittel (B) - bezogen auf das Gesamtgewicht des Mittels (B) - ein oder mehrere Säuren der Formel (I) in einer Gesamtmenge von 0,1 bis 10,0 Gew.-%, bevorzugt von 0,3 bis 8,5 Gew.-%, weiter bevorzugt von 0,5 bis 7,5 Gew.-% und ganz besonders bevorzugt von 0,7 bis 6,5 Gew.-% enthält.

## Claims

1. A method for lightening hair, comprising the following steps in the indicated order
(1) mixing an agent (A) with an agent (C) and/or an agent (D), wherein
- agent (A) contains hydrogen peroxide, and
- agent (C) contains at least one persulfate from the group consisting of ammonium persulfate, potassium persulfate and/or sodium persulfate, and
- agent (D) contains at least one alkalizing agent from the group consisting of ammonia, 2-aminoethane-1-ol, 2-amino-2-methylpropane-1-ol, 2-amino-2-methylpropane-1,3-diol, L-arginine, D-arginine, D/L-arginine, L-lysine, D-lysine and D/L-lysine,
(2) applying the mixture prepared in step (1) to the hair,
(3) allowing the mixture applied in step (2) to act on the hair,
(4) optionally rinsing the hair,
(5) applying an agent (B) to the hair, wherein agent (B) contains at least one acid of formula (I), 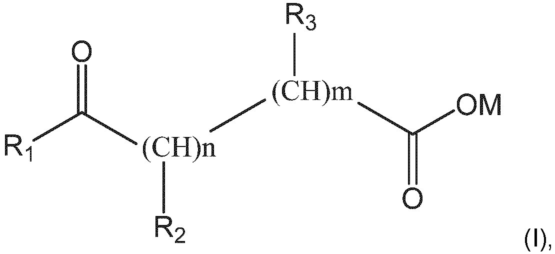 where
R₁ represents a C₁-C₆ alkyl group,
R₂, R₃ represent, independently of one another, a hydrogen atom, a C₁-C₆ alkyl group, a C₂-C₆ alkyl group,
M represents a hydrogen atom or an equivalent of a monovalent or polyvalent cation,
n represents the number 1 or 2, and
m represents the number 0 or 1,
and wherein agent (B) contains water and has a pH of 1.8 to 3.0.

2. The method according to claim 1, **characterized in that** agent (A), based on the total weight of agent (A), contains 0.1 to 12.5 wt.%, preferably 1.5 to 12.5 wt.%, more preferably 4.5 to 12.5 wt.% and very particularly preferably 6.5 to 12.5 wt.%, hydrogen peroxide.

3. The method according to one of claims 1 to 2, **characterized in that** agent (B) contains at least one acid of formula (I), where
R₁ represents a methyl group, an ethyl group or a propyl group, and preferably a methyl group.

4. The method according to one of claims 1 to 3, **characterized in that** agent (B) contains at least one acid of formula (I), where
M represents a hydrogen atom.

5. The method according to one of claims 1 to 4, **characterized in that** agent (B) contains at least one acid of formula (I) which is selected from the group consisting of 4-oxopentanoic acid (levulinic acid), 4-oxohexanoic acid, 2-methyl-4-oxo-pentanoic acid and/or 3-methyl-4-oxo-pentanoic acid.

6. The method according to one of claims 1 to 5, **characterized in that** agent (B), based on the total weight of the agent (B), contains one or more acids of formula (I) in a total amount of 0.1 to 10.0 wt.%, preferably 0.3 to 8.5 wt.%, more preferably 0.5 to 7.5 wt.%, and very particularly preferably 0.7 to 6.5 wt.%.

## Revendications

1. Procédé permettant l'éclaircissement de cheveux, comprenant les étapes suivantes dans l'ordre indiqué
(1) mélange d'un agent (A) avec un agent (C) et/ou un agent (D), dans lequel
- l'agent (A) contient du peroxyde d'hydrogène, et
- l'agent (C) contient au moins un persulfate choisi dans le groupe constitué de persulfate d'ammonium, persulfate de potassium et/ou persulfate de sodium, et
- l'agent (D) contient au moins un agent alcalinisant choisi dans le groupe constitué d'ammoniac, 2-aminoéthan-1-ol, 2-amino-2-méthylpropan-1-ol, 2-amino-2-méthyl-propane-1,3-diol, L-arginine, D-arginine, D/L-arginine, L-lysine, D-lysine et D/L-lysine,
(2) application du mélange préparé à l'étape (1) sur les cheveux,
(3) attente de l'action du mélange appliqué à l'étape (2) sur les cheveux,
(4) éventuellement, rinçage des cheveux,
(5) application d'un agent (B) sur les cheveux, dans lequel l'agent (B) contient au moins un acide de formule (I), 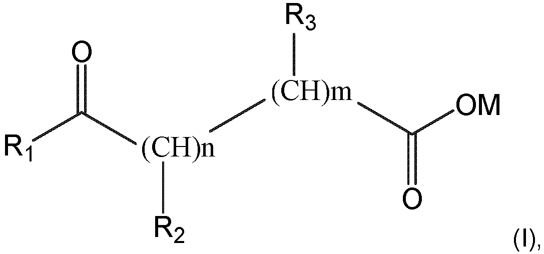 où
R₁ représente un groupe alkyle en C₁-C₆,
R₂, R₃ représentent, indépendamment l'un de l'autre, un atome d'hydrogène, un groupe alkyle en C₁-C₆, un groupe alcényle en C₂-C₆,
M représente un atome d'hydrogène ou un équivalent d'un cation monovalent ou polyvalent,
n représente le nombre 1 ou 2, et
m représente le nombre 0 ou 1,
et dans lequel l'agent (B) contient de l'eau et a un pH allant de 1,8 à 3,0.

2. Procédé selon la revendication 1, **caractérisé en ce que** l'agent (A) contient, par rapport au poids total de l'agent (A), 0,1 à 12,5 % en poids, de préférence 1,5 à 12,5 % en poids, plus préférablement 4,5 à 12,5 % en poids et de manière très particulièrement préférée 6,5 à 12,5 % en poids de peroxyde d'hydrogène.

3. Procédé selon l'une des revendications 1 à 2, **caractérisé en ce que** l'agent (B) contient au moins un acide de formule (I), où
R₁ représente un groupe méthyle, un groupe éthyle ou un groupe propyle, et de préférence un groupe méthyle.

4. Procédé selon l'une des revendications 1 à 3, **caractérisé en ce que** l'agent (B) contient au moins un acide de formule (I), où
M représente un atome d'hydrogène.

5. Procédé selon l'une des revendications 1 à 4, **caractérisé en ce que** l'agent (B) contient au moins un acide de formule (I) choisi dans le groupe constitué d'acide 4-oxopentanoïque (acide lévulinique), acide 4-oxohexanoïque, acide 2-méthyl-4-oxo-pentanoïque et/ou acide 3-méthyl-4-oxo-pentanoïque.

6. Procédé selon l'une des revendications 1 à 5, **caractérisé en ce que** l'agent (B) contient, par rapport au poids total de l'agent (B), un ou plusieurs acides de formule (I) en une quantité totale allant de 0,1 à 10,0 % en poids, de préférence de 0,3 à 8,5 % en poids, plus préférablement de 0,5 à 7,5 % en poids et de manière très particulièrement préférée de 0,7 à 6,5 % en poids.
